# EUROPEAN PATENT SPECIFICATION

(11) **EP 4 294 285 B1**
(45) Date of publication and mention of the grant of the patent: **06.05.2026**
(21) Application number: 22707548.8
(22) Date of filing: 08.02.2022
(51) Int. Cl.: A61B 17/115, A61B 17/00

(54) **SURGICAL STAPLING DEVICE INCLUDING A HYDRAULIC STAPLE FORMATION MECHANISM**
CHIRURGISCHE KLAMMERVORRICHTUNG MIT EINEM HYDRAULISCHEN KLAMMERBILDUNGSMECHANISMUS
DISPOSITIF D'AGRAFAGE CHIRURGICAL COMPRENANT UN MÉCANISME DE FORMATION D'AGRAFES HYDRAULIQUES

(30) Priority: 16.02.2021 US 202163149845 P; 07.12.2021 US 202117544313
(43) Date of publication of application: 27.12.2023
(73) Proprietor: Covidien LP, Mansfield, MA 02048 (US)
(72) Inventor: NICHOLAS, David A., Trumbull, Connecticut 06611 (US)
(74) Representative: Zimmermann & Partner Patentanwälte mbB
(86) International application number: PCT/US2022/015599
(87) International publication number: WO 2022/177774

(56) References cited:
- EP-A2- 3 243 447
- WO-A2-2014/008289
- US-A- 5 395 030

## Description

### CROSS-REFERENCE TO RELATED APPLICATION

This application claims the benefit of and priority to U.S. Provisional Patent Application Serial No. 63/149,845, filed on February 16, 2021, the entire disclosure of which is referenced herein.

### BACKGROUND

### Technical Field

The disclosure relates to surgical instruments and, more particularly, to an endoscopic stapling device with a flexible shaft that supports an end effector.

### Background of Related Art

Surgical stapling devices for performing surgical procedures endoscopically are well known and are commonly used to reduce patient trauma and shorten patient recovery times. Typically, an endoscopic stapling device includes a handle assembly, a rigid elongate body that extends distally from the handle assembly, and an end effector including a tool assembly that is supported on a distal portion of the elongate body. The handle assembly is coupled to the end effector by drive mechanisms that extend through the elongate body and allow a clinician to control operation of the end effector remotely via the handle assembly.

Surgical stapling devices for endoscopic use are available in a variety of configurations including linear and circular. Circular stapling devices are commonly used to perform anastomoses after resections of the large bowel, i.e., colectomies. In a large percentage of colectomy procedures, the portion of the colon that must be resected is in the ascending colon or the transverse colon which cannot be easily accessed by a circular stapling device having a rigid shaft. As such, these procedures are typically performed during an open colectomy procedure which result in increased patient trauma and recovery time.

US 5 395 030 discloses a surgical device for stapling and fastening body tissues, which comprises a staple-applying section containing a plurality of staples, a staple pusher incorporated in the staple-applying section, for applying the staples, an anvil section for deforming the staples, thereby to staple the body tissues together, an operation section for operating the staple pusher and the anvil section, an observation window located near the anvil section, and an ocular section for displaying images supplied from the observation window.

A continuing need exists in the medical arts for a stapling device having a flexible shaft for accessing a surgical site.

### SUMMARY

The invention is defined by the appended claims. In accordance with the disclosure, a shell assembly includes a shell housing defining a cavity, a plurality of staples, a staple cartridge supported on the shell housing, a pusher assembly positioned within the cavity of the shell housing, and a hydraulic piston configured to engage the pusher assembly to impart axial displacement to the pusher assembly and form a fluid tight seal against the shell housing. The staple cartridge defines slots that receive the plurality of staples. The pusher assembly includes a plurality of pushers arranged in an annular configuration to eject the plurality of staples from the staple cartridge. Supply of a fluid into the cavity of the shell housing advances the hydraulic piston, which, in turn, advances the pusher assembly to eject the plurality of staples from the staple cartridge.

In an aspect, the shell housing may include an annular guide in the cavity that defines a bore therethrough.

In another aspect, the shell assembly may further include an annular knife supported on the hydraulic piston for concomitant axial displacement therewith.

In yet another aspect, the hydraulic piston may define inner and outer circumferential grooves configured to receive respective inner and outer O-rings.

In still yet another aspect, the inner O-ring may be configured to engage the annular guide of the shell housing.

In still yet another aspect, the shell housing may include a clamp gear rotatably supported therein.

In still yet another aspect, the clamp gear may be aligned with the annular guide of the shell housing.

In an aspect, a proximal portion of the shell housing may include an inner lip defining a cutout to rotatably support a clamp input gear such that the clamp input gear operatively engages the clamp gear.

In another aspect, the inner lip may define a bore in communication with the cavity of the shell housing.

In accordance with another aspect of the disclosure, a surgical stapling device includes an adapter assembly and a tool assembly. The adapter assembly includes a flexible outer tube, a first drive assembly extending through the flexible outer tube, a second drive assembly extending through the flexible outer tube, and a third drive assembly. The first drive assembly includes a flexible approximation link and an anvil retainer secured to the flexible approximation link. The second drive assembly includes a flexible drive shaft and an input gear secured to the flexible drive shaft. The third drive assembly includes a hydraulic cylinder disposed in the flexible outer tube and a first piston movable within the hydraulic cylinder. The tool assembly is secured to a distal portion of the flexible outer tube and includes an anvil assembly and a shell assembly. The shell assembly includes a housing, a plurality of staples, a clamp gear supported within the housing, a staple cartridge, a pusher assembly, and a second piston. The anvil assembly includes an anvil head and an anvil shaft secured to the anvil head. The clamp gear is operatively engaged with the input gear of the second drive assembly. The staple cartridge is supported on the shell assembly. The staple cartridge defines staple receiving slots that receive the plurality of staples. The pusher assembly is positioned within the shell housing and includes a plurality of pushers to eject the plurality of staples from the staple cartridge. The second piston movably engages the shell housing of the shell assembly in a sealing relation and operatively engages the pusher assembly. Activation of the first drive assembly through a first clamping stage retracts the flexible articulation link to move the anvil retainer proximally to move the anvil assembly from an open position to a partially clamped position in which the anvil retainer is operatively engaged with the clamp gear. Activation of the second drive assembly through a second clamping stage moves the anvil retainer farther proximally to move the anvil assembly from the partially clamped position to a fully clamped position. Activation of the third drive assembly advances the first piston to direct the fluid into the housing of the shell assembly to advance the second piston, thereby advancing the pusher assembly.

In an aspect, the anvil shaft may have a threaded outer portion configured to be in registration with the clamp gear when the anvil assembly is in the partially retracted position.

In another aspect, the anvil shaft may be releasably coupled to the anvil retainer.

In yet another aspect, the surgical stapling device may further include a handle assembly. The proximal portion of the adapter assembly may be coupled to the handle assembly.

In still yet another aspect, the adapter assembly may include a flexible fluid supply tube interconnecting the hydraulic cylinder and the housing of the shell assembly.

In accordance with still yet another aspect of the disclosure, a surgical stapling device includes an adapter assembly and a tool assembly. The adapter assembly includes a flexible outer tube, a first drive assembly extending through the flexible outer tube, a second drive assembly extending through the flexible outer tube, and a third drive assembly. The first drive assembly includes a flexible approximation link and an anvil retainer secured to a distal portion of the flexible approximation link. The second drive assembly includes a flexible drive shaft and an input gear secured to a distal portion of the flexible drive shaft. The third drive assembly includes a hydraulic cylinder, a first piston, and a flexible tube in fluid communication with the hydraulic cylinder. The tool assembly is secured to a distal portion of the flexible outer tube and includes an anvil assembly and a shell assembly. The anvil assembly includes an anvil head and an anvil shaft secured to the anvil head. The shell assembly includes a shell housing, a staple cartridge, a pusher assembly, a second piston, and a clamp gear. The shell housing defines a cavity in fluid communication with the flexible tube of the third drive assembly. The staple cartridge is supported on the shell housing and defines staple receiving slots that receive a plurality of staples. The pusher assembly is configured to eject the plurality of staples from the staple cartridge. The pusher assembly is positioned within the cavity of the shell housing and includes a plurality of pushers arranged in an annular configuration. The plurality of pushers is transitionable from a retracted position to an advanced position to eject the plurality of staples from the staple cartridge. The second piston is movable within the cavity and operatively engages the pusher assembly. The clamp gear is rotatably supported within the shell housing of the shell assembly and is engaged with the input gear of the second drive assembly. Activation of the first drive assembly through a first clamping stage retracts the flexible approximation link to move the anvil retainer proximally to move the anvil assembly from an open position to a partially clamped position, in which, the anvil shaft is operatively engaged with the clamp gear. Activation of the second drive assembly through a second clamping stage moves the anvil retainer farther proximally to move the anvil assembly from the partially clamped position to a fully clamped position. Activation of the third drive assembly advances the first piston in the hydraulic cylinder to direct a fluid into the cavity of the shell housing through the flexible tube, thereby advancing the pusher assembly.

### BRIEF DESCRIPTION OF THE DRAWINGS

Various aspects of the disclosure are described hereinbelow with reference to the drawings, which are incorporated and constitute a part of this specification, wherein:
FIG. 1 is a perspective view of a surgical stapling device in accordance with the disclosure;
FIG. 2 is a perspective view of an adapter assembly and tool assembly of the surgical stapling device of FIG. 1,
FIG. 3 is an exploded perspective view of the tool assembly of the adapter assembly of FIG. 2 with parts separated;
FIG. 4 is an enlarged view of the indicated area of detail of FIG. 3,
FIG. 5 is a rear perspective view of a shell housing of FIG. 4;
FIG. 6 is an enlarged view of the indicated area of detail of FIG. 3;
FIG. 7 is a rear perspective view of a piston of FIG. 6;
FIG. 8 is a perspective view of the tool assembly of FIG. 2;
FIG. 9 is a perspective view of the tool assembly of FIG. 8 with the shell housing removed;
FIG. 10 is a side cross-sectional view taken along section line 10-10 of FIG. 1,
FIG. 11 is a partial side cross-sectional view of the adapter assembly of FIG. 10, illustrating retraction of the anvil assembly;
FIG. 12 is a partial side cross-sectional view of the adapter assembly of FIG. 10, illustrating close approximation of an anvil assembly;
FIG. 13 is a partial perspective view of the adapter assembly of FIG. 2, illustrating close approximation of the anvil assembly;
FIG. 14 is a side cross-sectional view of the adapter assembly of FIG. 13 taken along section line 13-13;
FIG. 15 is an enlarged view of the indicated area of detail of FIG. 14;
FIG. 16 is a side cross-sectional view of the adapter assembly of FIG. 14, illustrating actuation of a firing mechanism of FIG. 14; and
FIG. 17 is an enlarged view of the indicated area of detail of FIG. 16.

### DETAILED DESCRIPTION

The disclosed surgical instrument is described in detail with reference to the drawings, in which like reference numerals designate identical or corresponding elements in each of the several views. As used herein, the term "distal," as is conventional, will refer to that portion of the instrument, apparatus, device, or component thereof which is farther from the user, while the term "proximal" will refer to that portion of the instrument, apparatus, device, or component thereof which is closer to the user. As used herein, the terms parallel and perpendicular are understood to include relative configurations that are substantially parallel and substantially perpendicular up to about + or - 10 degrees from true parallel and true perpendicular. In the following description, well-known functions or constructions are not described in detail to avoid obscuring the disclosure in unnecessary detail.

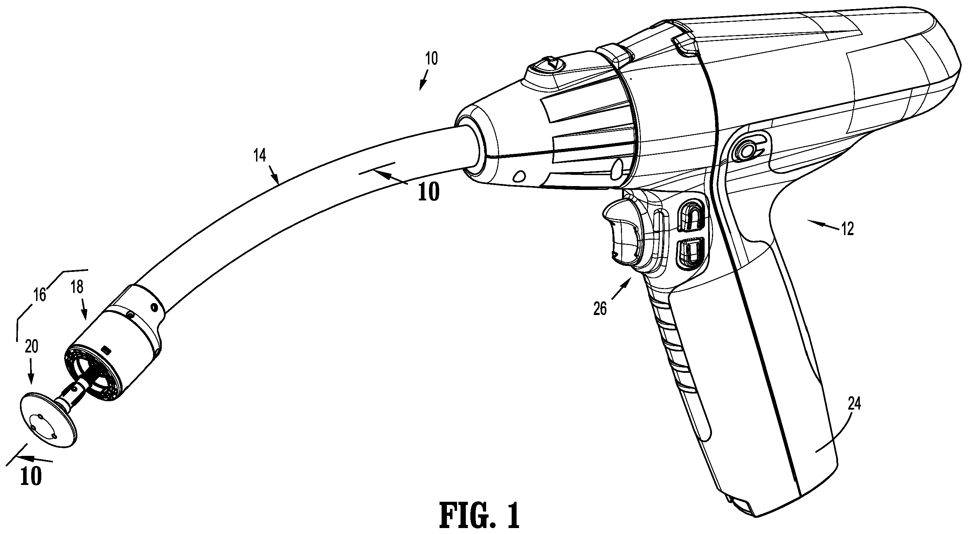

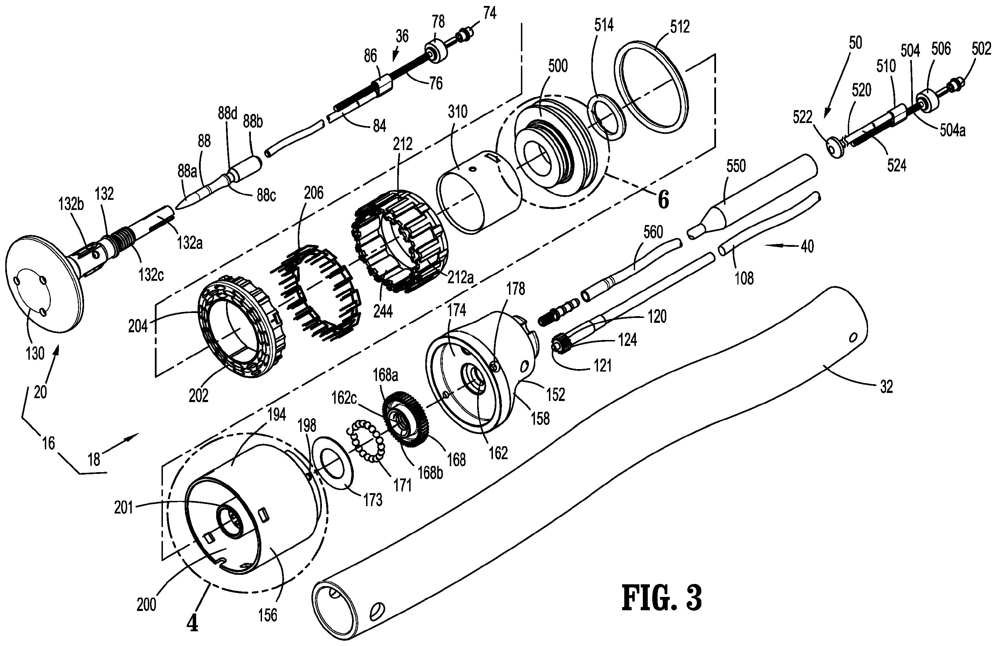

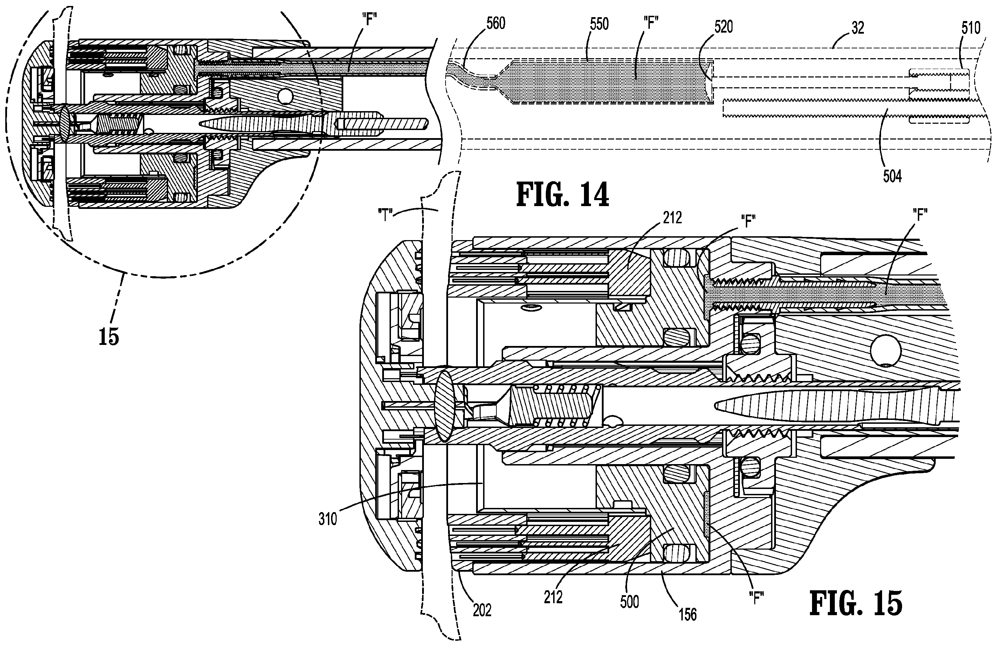

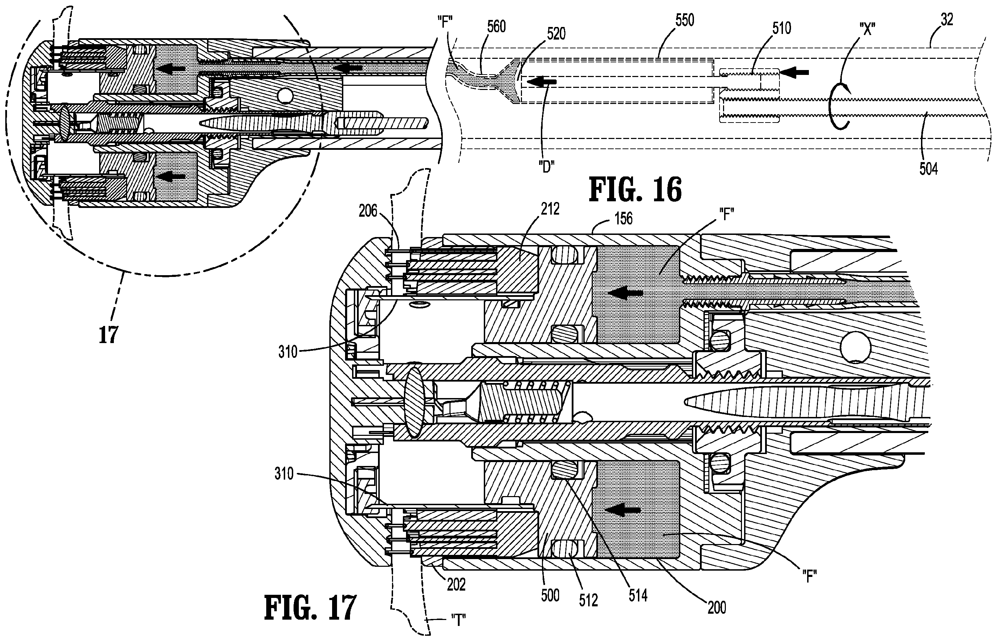

With reference to FIG. 1, a surgical instrument in accordance with the disclosure is generally designated as 10 and is in the form of a powered stapling device used to create an anastomosis between two anatomical lumens such as, e.g., colon or esophagus. The surgical instrument 10 provides flexibility to facilitate introduction and positioning of the surgical instrument 10 into a body cavity through natural orifices or incisions around the contours and curvatures of the anatomy within the body cavity, as will be described. The surgical instrument 10 includes a powered handheld electromechanical device, shown generally as a handle assembly 12, an adapter assembly 14, and a tool assembly 16. The tool assembly 16 includes a shell assembly 18 and an anvil assembly 20 that is supported for movement in relation to the shell assembly 18 between an open position (FIG. 1) and a clamped position (FIG. 15). The handle assembly 12 includes a stationary grip 24 that supports actuation buttons 26 for controlling operation of various functions of the surgical instrument 10 including approximation of the shell and anvil assemblies 18, 20, firing of staples 206 (FIG. 3) from the shell assembly 18, and cutting or coring of tissue "T" (FIG. 17).

The surgical instrument 10 is an electrically powered stapling device. As such, the handle assembly 12 may support a motor, control circuitry, and a battery or battery pack (not shown) for driving various mechanisms of the adapter assembly 14 to facilitate approximation of the shell and anvil assemblies 18, 20, firing of staples 206 from the shell assembly 18, and cutting or coring of tissue "T" (FIG. 17). Examples of electrically powered stapling devices including a handle assembly suitable for use with the disclosed surgical instrument 10 can be found in U.S. Patent Nos. 9,055,943 (the '943 Patent), 9,023,014 (the '014 Patent), and U.S. Publication Nos. 2018/0125-495, and 2017/0340351.

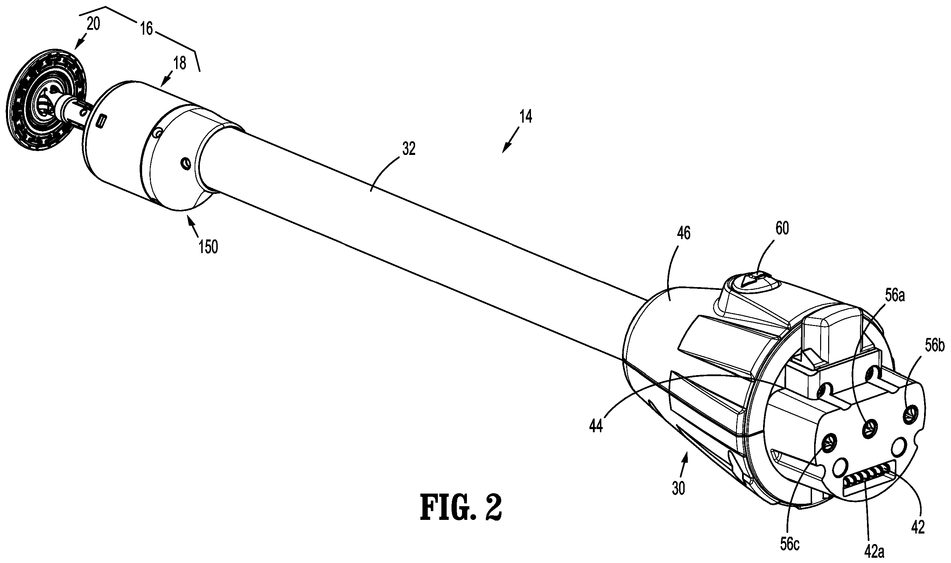

FIGS. 2 and 3 illustrate the adapter assembly 14 that includes a proximal housing assembly 30, a flexible outer tube 32, a first drive assembly 36, a second drive assembly 40, and a third drive assembly 50. The proximal housing assembly 30 includes a hub 44, a rotation knob 46 that is rotatably supported on the hub 44, and an electrical coupling member 42. The hub 44 defines through bores 56a, 56b, and 56c that receive proximal ends of the first, second, and third drive assemblies 34, 40, 50 of the adapter assembly 14. The electrical coupling member 42 includes proximally extending contacts 42a that are positioned to engage contacts in the handle assembly 12 (FIG. 1) when the adapter assembly 14 is coupled to the handle assembly 12 to electrically couple the control circuitry (not shown) in the handle assembly 12 to sensors (not shown) in the adapter assembly 14 and/or tool assembly 16 to facilitate control of the operation of the surgical instrument 10 as known in the art. The hub 44 also supports an adapter release button 60 which can be depressed to facilitate separation of the adapter assembly 14 from the handle assembly 12.

FIG. 3 further illustrates the first drive assembly 36 including a drive connector 74, a threaded drive shaft 76, and a bearing 78. The bearing 78 is received within the through bore 56a (FIG. 2) of the hub 44 and defines a through bore that receives an unthreaded proximal end portion of the threaded drive shaft 76 such that the threaded drive shaft 76 is rotatably supported within the bearing 78. The drive connector 74 is fixedly secured to the proximal end of the threaded drive shaft 76 and defines a recess that receives a first drive member (not shown) of the handle assembly 12 (FIG. 1). Under such a configuration, the drive connector 74 imparts rotational input from the handle assembly 12 to the threaded drive shaft 76 within the rotation knob 46 (FIG. 2).

The threaded drive shaft 76 of the first drive assembly 36 is coupled to a flexible approximation link 84 by a threaded coupling member 86. The threaded coupling member 86 defines a threaded bore that receives the threaded drive shaft 76. When the threaded drive shaft 76 is rotated within the rotation knob 46 (FIG. 2) and the flexible outer tube 32 (FIG. 2), the threaded coupling member 86 translates along the threaded drive shaft 76 to cause axial displacement of the flexible approximation link 84 within the flexible outer tube 32 between an advanced position in which the threaded coupling member 86 is positioned on a distal portion of the threaded drive shaft 76 and a retracted position in which the threaded coupling member 86 is positioned on a proximal portion of the threaded drive shaft 76. The distal end of the flexible approximation link 84 is secured to an anvil retainer 88 that includes a distal trocar portion 88a and a proximal portion 88b. The proximal portion 88b of the anvil retainer 88 is coupled to the flexible approximation link 84 such that the anvil retainer 88 is movable with the flexible approximation link 84 between advanced and retracted positions.

Similar to the first drive assembly 36, the second drive assembly 40 includes a drive connector (not shown), a flexible drive shaft 108, and a bearing (not shown). The bearing is received within the through bore 56b (FIG. 2) defined in the hub 44 and defines a through bore that receives a proximal end of the flexible drive shaft 108 such that the flexible drive shaft 108 is rotatably supported within the bearing. The drive connector is fixedly secured to the proximal end of the flexible drive shaft 108 and defines a recess that receives a second drive member (not shown) of the handle assembly 12 (FIG. 1). The drive connector is rotatable to impart rotation to the flexible drive shaft 108 within the rotation knob 46 (FIG. 2) and outer tube 32 (FIG. 2). The flexible drive shaft 108 has a distal portion that supports a clamp input gear 120 that includes a cylindrical body portion having a protrusion 121 and a distal gear member 124. The clamp input gear 120 is fixedly secured to the flexible drive shaft 108 such that rotation of the flexible drive shaft 108 imparts concomitant rotation to the clamp input gear 120.

The third drive assembly 50 includes a drive connector 502, a drive shaft 504, a bearing 506, a piston assembly 520, a hydraulic cylinder 550, and a fluid supply tube 560. The bearing 506 is received within the through bore 56c (FIG. 2) defined in the hub 44 and defines a through bore that receives an unthreaded proximal end portion of the drive shaft 504 such that the drive shaft 504 is rotatably supported within the bearing 506. The drive connector 502 is fixedly secured to a proximal end of the drive shaft 504 and defines a recess that receives a third drive member (not shown) of the handle assembly 12 (FIG. 1). Under such a configuration, the drive connector 502 imparts concomitant rotation to the drive shaft 504 within the rotation knob 46 (FIG. 2).

The drive shaft 504 has a threaded portion 504a that is threadably coupled to a coupling member 510. In particular, the coupling member 510 defines a threaded bore that receives the threaded portion 504a of the drive shaft 504. The coupling member 510 is also coupled to the piston assembly 520 for movement as a single construct. The piston assembly 520 includes a piston head 522 and a piston shaft 524 connected to the coupling member 510. Under such a configuration, when the drive shaft 504 is rotated within the rotation knob 46 (FIG. 2) and the flexible outer tube 32 (FIG. 2), the coupling member 510 translates along the drive shaft 504 to cause axial displacement of the piston assembly 520 within the flexible outer tube 32 between an advanced position in which the piston head 522 is positioned at a distal end portion of the hydraulic cylinder 550 and a retracted position in which the piston head 522 is positioned at a proximal end portion of the hydraulic cylinder 550. The piston assembly 520 is received in the hydraulic cylinder 550 to displace a volume of fluid into and out of the hydraulic cylinder 550 during the transition between the advanced and retracted positions of the piston head 522. The fluid supply tube 560 is in communication with the hydraulic cylinder 550 and the shell assembly 18 to supply the fluid to the shell assembly 18 to actuate firing of staples 206 and cutting of tissue "T" (FIG. 17), as will be described below.

FIG. 3 further illustrates the tool assembly 16 of the surgical instrument 10 which includes the shell assembly 18 and the anvil assembly 20. The anvil assembly 20 includes an anvil head 130 and an anvil shaft 132. The anvil shaft 132 has a distal portion that is coupled to the anvil head 130 and a proximal portion that is adapted to be releasably coupled to the anvil retainer 88. In aspects of the disclosure, the proximal portion of the anvil shaft 132 includes resilient fingers 132a that define a longitudinal cavity (not shown) that receives the anvil retainer 88. When the anvil retainer 88 is inserted into the longitudinal cavity of the anvil shaft 132, the resilient fingers 132a of the anvil shaft 132 engage and move along an outwardly diverging surface 88c of the anvil retainer 88 and are deformed outwardly to facilitate passage of the anvil retainer 88 into the longitudinal cavity. When the anvil retainer 88 is received within the longitudinal cavity, the resilient fingers 132a of the anvil shaft 132 return to their nondeformed condition and engage an annular shoulder 88d of the anvil retainer 88 to releasably couple the anvil shaft 132 to the anvil retainer 88. The anvil shaft 132 also supports a plurality of longitudinal splines 132b that are spaced about the periphery of the anvil shaft 132. In some aspects of the disclosure, the anvil head 130 is pivotably coupled to the anvil shaft 132. For a more detailed description of an anvil assembly including a pivotable head assembly, see, e.g., U.S. Patent No. 6,945,444 ("the '444 Patent").

The shell assembly 18 includes a housing assembly 150 (FIG. 2) that includes a proximal housing 152 and a shell housing 156. The proximal housing 152 includes a body 158 configured to receive the first, second, and third drive assemblies 36, 40, 50. In particular, the proximal housing 152 defines a first through bore 162 centrally located along a longitudinal axis "X" of the shell assembly 18. The first through bore 162 receives the flexible approximation link 84 of the first drive assembly 36. The distal portion of the proximal housing 152 includes an annular extension 172 that defines a cavity 174.

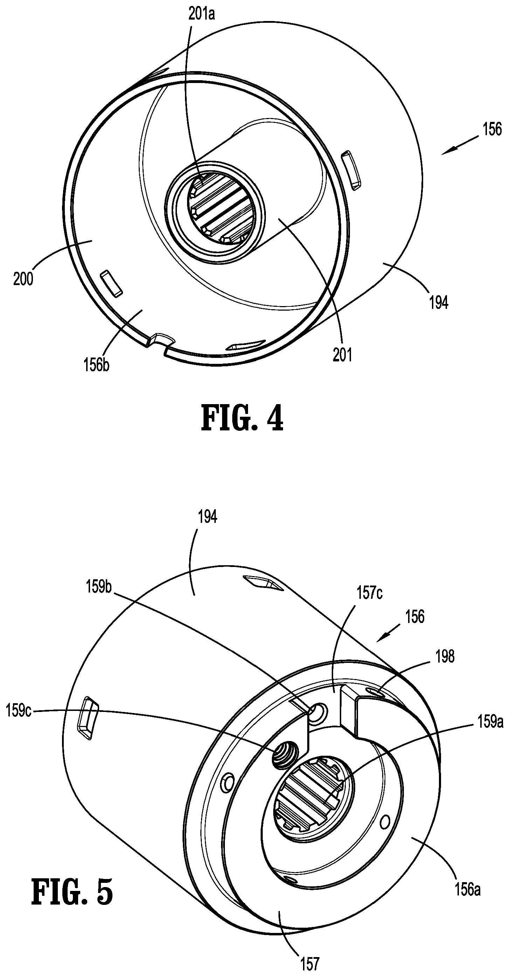

FIGS. 3-5 illustrate the shell housing 156 of the housing assembly 150 including a cylindrical body 194 that has a proximal portion 156a and a distal portion 156b. The proximal portion 156a of the cylindrical body 194 has an inner lip 157 (FIG. 5) that is received in the cavity 174 (FIG. 3), i.e., within the annular rim 172, of the proximal housing 152. In particular, the inner lip 157 defines a cutout 157c.

The proximal portion 156a of the shell housing 156 defines first, second, and third bores 159a, 159b, 159c. With brief reference to FIG. 3, the proximal portion 156a rotatably supports a clamp gear 168 in registration with the first bore 159a. The clamp gear 168 includes a cylindrical extension 168a. that is concentrically disposed with respect to the clamp gear 168. The cylindrical extension 168a defines a bore 168b that is in communication with the first through bore 162 of the proximal housing 152 and the first bore 159a of the shell housing 156. The clamp gear 168 supports a thrust bearing 171 and a thrust washer 173 about the cylindrical extension 168a. to facilitate rotation of the clamp gear 168. The shell housing 156 and the proximal housing 152 are fixedly secured together using screws (not shown) which are received through openings 198 in the shell housing 156 and openings 178 in the rim 172 of the proximal housing 152.

In this manner, the shell housing 156 extends distally from the proximal housing 152. The shell housing 156 defines an inner cavity 200, in which, the shell housing 156 includes an annular guide 201. The first bore 159a of the shell housing 156 extends through the annular guide 201. The second bore 159b is configured to receive a protrusion 121 of the clamp input gear 120 of the second drive assembly 40 to rotatably support the clamp input gear 120 within the cutout 157c of the shell housing 156. The third bore 159c is in communication with the inner cavity 200 and is connected to the fluid supply tube 560 (FIG. 3) of the third drive assembly 50. Further, the annular guide 201 has an inner surface including splines 201a to guide the complementary splines 132b of the anvil assembly 20.

The distal portion 156b of the shell housing 156 supports an annular staple cartridge 202 that defines staple receiving slots 204. Each of the staple receiving slots 204 receives a staple 206. In one aspect of the disclosure, the staple receiving slots 204 are formed in one or more circular rows in the annular staple cartridge 202. In addition, a pusher assembly 212 is supported in the inner cavity 200 of the shell housing 156 adjacent the annular staple cartridge 202. The pusher assembly 212 includes a plurality of pushers 212a that is coupled together to form an annular configuration. The shell assembly 18 further includes an annular knife 310 disposed radially inward of the pusher assembly 212. The annular knife 310 and the pusher assembly 212 are operatively coupled to a piston 500 disposed within the inner cavity 200 of the shell housing 156.

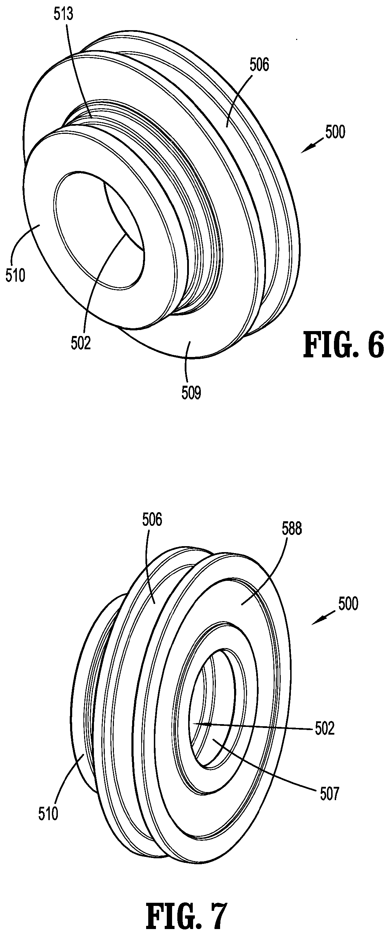

In particular, FIGS. 6 and 7 illustrate the piston 500 having an annular configuration defining a bore 502 therethrough. The piston 500 further includes an annular extension 510 configured to secure the annular knife 3 10 thereto. In particular, the annular extension 510 defines a circular groove 513 (FIG. 6) to receive a radial tang (not shown) of the annular knife 310 in order to enhance securement therewith. The piston 500 further includes a flange portion 509 configured to engage the pusher assembly 212 (FIG. 3). The piston 500 defines an outer circumferential groove 506 on an outer surface of the piston 500 and an inner circumferential groove 507 on an inner surface of the piston 500. First and second o-rings 512, 514 are received in the outer and inner circumferential grooves 506, 507, respectively, to thereby form a fluid tight seal against the shell housing 156. The proximal portion of the piston 500 defines a recess 588 to contain the fluid "F" (FIG. 15) therein.

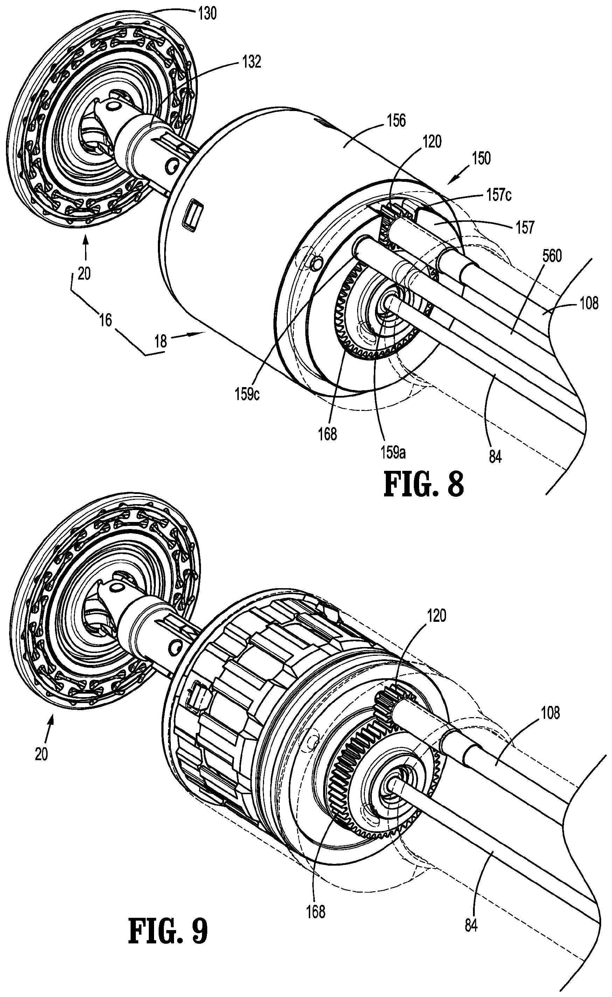

FIGS. 8 and 9, illustrate the flexible approximation link 84 of the first drive assembly 36 extending through the first bore 159a of the shell housing 156. The clamp input gear 120 of the second drive assembly 40 is rotatably supported in the cutout 157c of the inner lip 157 of the shell housing 156. The clamp input gear 120 engages the clamp gear 168 to impart rotation to the clamp gear 168. The clamp gear 168 has a threaded inner surface 162c (FIG. 3) that is configured to threadably engage a threaded portion 132c (FIG. 3) of the anvil shaft 132, as will be described below. The fluid supply tube 560 is connected to the third bore 159c of the shell housing 156 to supply a hydraulic fluid to the inner cavity 200 (FIG. 4) of the shell housing 156, thereby enabling axial displacement of the piston 500 to effect firing of staples and cutting of tissue, as will be described below.

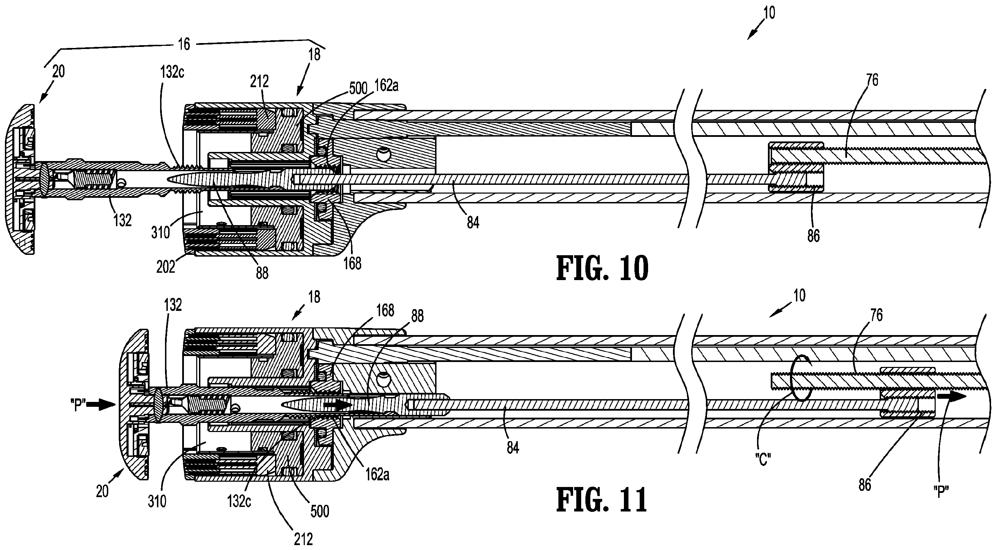

FIGS. 10 and 11 illustrate retraction of the anvil assembly 20 relative to the shell assembly 18. In particular, FIG. 10 illustrates the surgical instrument 10 in a pre-fired position with the anvil assembly 20 coupled to the anvil retainer 88 and the tool assembly 16 in the open position. In this position, a distal end of the threaded drive shaft 76 of the first drive assembly 36 (FIG. 3) is received in the threaded coupling member 86 and the flexible approximation link 84 of the first drive assembly 36 is in its advanced position such the anvil assembly 20 is spaced from the annular staple cartridge 202 of the shell assembly 18. In addition, the piston 500, the pusher assembly 212, and the annular knife 310 are in their retracted positions within the shell housing 156 of the shell assembly 18.

The drive connector 74 (FIG. 3) of the first drive assembly 36 receives a rotational input from an actuator (not shown) of the handle assembly 12 (FIG. 1). Rotational input provides rotation to the threaded drive shaft 76 in the direction of, e.g., an arrow "C" in FIG. 11, which, in turn, causes axial displacement of the threaded coupling member 86 in the direction of an arrow "P", relative to the threaded drive shaft 76. The anvil retainer 88 is coupled to the threaded coupling member 86 via the flexible approximation link 84. Under such a configuration, the anvil assembly 20 that is coupled to the anvil retainer 88 is transitionable between an advanced position (FIG. 10) and a retracted position (FIG. 11). In particular, when the anvil assembly 20 is in the retracted position, the threaded portion 132c of the anvil shaft 132 is placed in registration with the threaded inner surface 162a of the clamp gear 168.

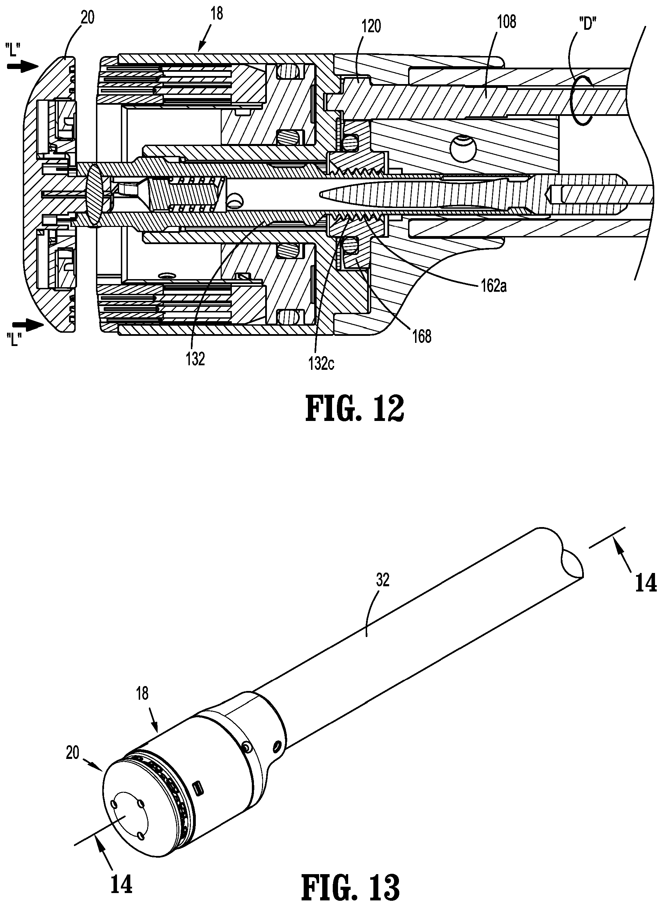

FIGS. 12 and 13 illustrate close approximation of the anvil assembly 20 relative to the shell assembly 18. Specifically, the surgical instrument 10 is actuated to move the tool assembly 16 through a second clamping stage to a fully clamped position. Close approximation of the anvil assembly 20 is effected when the anvil assembly 20 is in the retracted position through the use of the second drive assembly 40 (FIG. 3). When the anvil assembly 20 is in the retracted position, the threaded portion 132c of the anvil shaft 132 is in registration with the threaded inner surface 162a of the clamp gear 168. Under such a configuration, rotation of the flexible drive shaft 108 imparts concomitant rotation to the clamp input gear 120. The clamp input gear 120 engages the clamp gear 168 to impart rotation thereto. In this manner, rotation of the clamp input gear 120, e.g., in the direction of an arrow "D" (FIG. 12) causes close approximation of the anvil assembly 20 in the direction of arrows "L". Under such a configuration, the clamping forces to move the tool assembly 16 from the partially clamped position to the fully clamped position during the second clamping stage are contained within the housing assembly 150 of the shell assembly 18 and are not distributed through flexible outer tube 32 of the adapter assembly 14.

FIGS. 14-16 illustrate a firing mechanism of the surgical instrument 10. To fire the staples 206 (FIG. 3) from the annular staple cartridge 202, the handle assembly 12 is actuated to activate the third drive assembly 50 (FIG. 3). The drive shaft 504 of the third drive assembly 50 receives rotational input from an actuator of the handle assembly 12. Rotation of the drive shaft 504 causes axial displacement of the coupling member 510, which, in turn, imparts axial displacement to the piston assembly 520. For example, rotation of the drive shaft 504 in the direction of an arrow "X" (FIG. 16) causes axial displacement of the piston assembly 520 in the direction of an arrow "D" which directs the hydraulic fluid "F" in the hydraulic cylinder 550 towards third bore 159c (FIG. 5) of the shell housing 156 via the fluid supply tube 560. As the piston assembly 520 travels distally in the hydraulic cylinder 550, the hydraulic fluid "F" is displaced into the inner cavity 200 of the shell housing 156, thereby advancing the piston 500 in the inner cavity 200.

The piston 500 imparts axial displacement to the pusher assembly 212 and the annular knife 310. The pushers 212a advance through the staple receiving slots 204 of the annular staple cartridge 202 to eject the staples 206 from the annular staple cartridge 202 (FIG. 3). In addition, the annular knife 310 is also advanced to cut tissue "T". Through the use of the hydraulic fluid "F" supplied through the fluid supply tube 560, the need for a rigid or a semi-rigid member for the firing mechanism is eliminated. In this manner, a maximum shaft flexibility of the flexible outer tube 32 is effected, while generating adequate firing forces and minimizing the distal length of the device. The flexible outer tube 32 may provide the maximum flexibility to accommodate the contours and curvatures of the anatomical structures of a patient.

Any of the components described herein may be fabricated from either metals, plastics, resins, composites, or the like taking into consideration strength, durability, wearability, weight, resistance to corrosion, ease of manufacturing, cost of manufacturing, and the like. It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

Persons skilled in the art will understand that the devices and methods specifically described herein and illustrated in the accompanying drawings are non-limiting exemplary aspects of the disclosure. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

## Claims

1. A shell assembly (18) comprising:
a shell housing (156) defining a cavity (174);
a plurality of staples (206);
a staple cartridge (202) supported on the shell housing, the staple cartridge defining slots (204) that receive the plurality of staples;
a pusher assembly (212) positioned within the cavity of the shell housing and including a plurality of pushers (212a) arranged in an annular configuration to eject the plurality of staples from the staple cartridge; and
a hydraulic piston (500) configured to engage the pusher assembly to impart axial displacement to the pusher assembly and form a fluid tight seal against the shell housing,
wherein supply of a fluid into the cavity of the shell housing advances the hydraulic piston, which, in turn, advances the pusher assembly to eject the plurality of staples from the staple cartridge, wherein the shell housing includes an annular guide (201) in the cavity, the annular guide defining a bore (159a) therethrough ;
**characterised in that** the shell housing includes a clamp gear (168) rotatably supported therein.

2. The shell assembly according to claim 1, further comprising an annular knife (310) supported on the hydraulic piston for concomitant axial displacement therewith.

3. The shell assembly according to claim 1 or 2, wherein the hydraulic piston defines inner and outer circumferential grooves (506, 507) that receive respective inner and outer O-rings (512, 514).

4. The shell assembly according to claim 3, wherein the inner O-ring is configured to engage the annular guide of the shell housing.

5. The shell assembly according to claim 1, wherein the clamp gear is aligned with the annular guide of the shell housing.

6. The shell assembly according to claim 5, wherein a proximal portion of the shell housing includes an inner lip (157) defining a cutout to rotatably support a clamp input gear (120) such that the clamp input gear operatively engages the clamp gear.

7. The shell assembly according to claim 6, wherein the inner lip defines a bore in communication with the cavity of the shell housing.

8. A surgical stapling device comprising:
an adapter assembly (14) including:
a flexible outer tube (32);
a first drive assembly (36) extending through the flexible outer tube, the first drive assembly including a flexible approximation link (84) and an anvil retainer (88) secured to the flexible approximation link;
a second drive assembly (40) extending through the flexible outer tube, the second drive assembly including a flexible drive shaft (108) and an input gear (120) secured to the flexible drive shaft; and
a third drive assembly (50) including:
a hydraulic cylinder (550) disposed in the flexible outer tube; and
a first piston (520) movable within the hydraulic cylinder; and
a tool assembly (16) secured to a distal portion of the flexible outer tube, the tool assembly including:
an anvil assembly (20) including an anvil head (130) and an anvil shaft (132) secured to the anvil head; and
a shell assembly as claimed in claim 1,
wherein the clamp gear is supported within the housing of the shell assembly, the clamp gear operatively engaged with the input gear of the second drive assembly;
wherein activation of the first drive assembly through a first clamping stage retracts the flexible articulation link to move the anvil retainer proximally to move the anvil assembly from an open position to a partially clamped position in which the anvil retainer is operatively engaged with the clamp gear,
activation of the second drive assembly through a second clamping stage moves the anvil retainer farther proximally to move the anvil assembly from the partially clamped position to a fully clamped position, and
activation of the third drive assembly advances the first piston to direct the fluid into the housing of the shell assembly to advance the second piston, thereby advancing the pusher assembly.

9. The surgical stapling device according to claim 8, wherein the anvil shaft has a threaded outer portion in registration with the clamp gear when the anvil assembly is in the partially retracted position.

10. The surgical stapling device according to claim 9, wherein the anvil shaft is releasably coupled to the anvil retainer.

11. The surgical stapling device according to claim 8, further including a handle assembly (12), the adapter assembly having a proximal portion coupled to the handle assembly.

12. The surgical stapling device according to claim 8, wherein the adapter assembly includes a flexible fluid supply tube (560) interconnecting the hydraulic cylinder and the housing of the shell assembly such that the hydraulic cylinder and the housing of the shell assembly are in fluid communication.

13. A surgical stapling device comprising:
an adapter assembly (14) including:
a flexible outer tube (32);
a first drive assembly (36) extending through the flexible outer tube, the first drive assembly including a flexible approximation link (84) and an anvil retainer (88) secured to a distal portion of the flexible approximation link;
a second drive assembly (40) extending through the flexible outer tube, the second drive assembly including a flexible drive shaft (108) and an input gear (120) secured to a distal portion of the flexible drive shaft; and
a third drive assembly (50) including a hydraulic cylinder (550), a first piston (520), and a flexible tube (560) in fluid communication with the hydraulic cylinder; and
a tool assembly (16) secured to a distal portion of the flexible outer tube, the tool assembly including:
an anvil assembly (20) including an anvil head (130) and an anvil shaft (132) secured to the anvil head; and
a shell assembly as claimed in claim 1, wherein
the cavity is in fluid communication with the flexible tube of the third drive assembly;
the clamp gear is engaged with the input gear of the second drive assembly,
wherein activation of the first drive assembly through a first clamping stage retracts the flexible approximation link to move the anvil retainer proximally to move the anvil assembly from an open position to a partially clamped position, in which, the anvil shaft is operatively engaged with the clamp gear,
wherein activation of the second drive assembly through a second clamping stage moves the anvil retainer farther proximally to move the anvil assembly from the partially clamped position to a fully clamped position, and
wherein activation of the third drive assembly advances the first piston in the hydraulic cylinder to direct a fluid into the cavity of the shell housing through the flexible tube, thereby advancing the pusher assembly.

14. The stapling device according to claim 13, wherein the tool assembly further includes an annular knife (310) supported on the hydraulic piston.

15. The stapling device according to claim 14, wherein the clamp gear has a threaded bore configured to threadably engage a threaded outer portion of the anvil shaft.

## Patentansprüche

1. Schalenbaugruppe (18), umfassend:
ein Schalengehäuse (156), das einen Hohlraum (174) definiert;
eine Vielzahl von Klammern (206);
ein Klammermagazin (202), das auf dem Schalengehäuse getragen wird, wobei das Klammermagazin Schlitze (204) definiert, die die Vielzahl von Klammern aufnehmen;
eine Schieberanordnung (212), die innerhalb des Hohlraums des Schalengehäuses angeordnet ist und eine Vielzahl von Schiebern (212a) aufweist, die in einer ringförmigen Konfiguration angeordnet sind, um die Vielzahl von Klammern aus dem Klammermagazin auszuwerfen; und
einen hydraulischen Kolben (500), der ausgelegt ist, die Schieberanordnung in Eingriff zu nehmen, um eine axiale Verschiebung auf die Schieberanordnung zu übertragen und eine flüssigkeitsdichte Abdichtung gegen das Schalengehäuse zu bilden,
wobei die Zufuhr eines Fluids in den Hohlraum des Schalengehäuses den hydraulischen Kolben vorschiebt, wodurch wiederum die Schieberanordnung vorgeschoben wird, um die Vielzahl von Klammern aus dem Klammermagazin auszuwerfen, wobei das Schalengehäuse eine ringförmige Führung (201) in dem Hohlraum aufweist, wobei die ringförmige Führung eine Bohrung (159a) dort hindurch definiert;
**dadurch gekennzeichnet, dass** das Schalengehäuse ein Klemmzahnrad (168) aufweist, das drehbar darin getragen wird.

2. Schalenbaugruppe nach Anspruch 1, ferner umfassend ein ringförmiges Messer (310), das zur gleichzeitigen axialen Verschiebung damit auf dem hydraulischen Kolben getragen wird.

3. Schalenbaugruppe nach Anspruch 1 oder 2, wobei der hydraulische Kolben ferner innere und äußere Umfangsnuten (506, 507) definiert, die jeweilige innere und äußere O-Ringe (512, 514) aufnehmen.

4. Schalenbaugruppe nach Anspruch 3, wobei der innere O-Ring ausgelegt ist, die ringförmige Führung des Schalengehäuses in Eingriff zu nehmen.

5. Schalenbaugruppe nach Anspruch 1, wobei das Klemmzahnrad zu der ringförmigen Führung des Schalengehäuses ausgerichtet ist.

6. Schalenbaugruppe nach Anspruch 5, wobei ein proximaler Abschnitt des Schalengehäuses eine innere Lippe (157) aufweist, die einen Ausschnitt zum drehbaren Unterstützen eines Klemm-Eingangszahnrads (120) definiert, so dass das Klemm-Eingangszahnrad das Klemmzahnrad funktionsfähig in Eingriff nimmt.

7. Schalenbaugruppe nach Anspruch 6, wobei die innere Lippe eine Bohrung definiert, die mit dem Hohlraum des Schalengehäuses in Verbindung steht.

8. Chirurgische Klammernahtvorrichtung, umfassend:
eine Adapteranordnung (14), die Folgendes aufweist:
einen flexiblen Außenschlauch (32);
eine erste Antriebsanordnung (36), die sich durch den flexiblen Außenschlauch erstreckt, wobei die erste Antriebsanordnung ein flexibles Annäherungsglied (84) und einen an dem flexiblen Annäherungsglied befestigten Ambosshalter (88) aufweist;
eine zweite Antriebsanordnung (40), die sich durch den flexiblen Außenschlauch erstreckt, wobei die zweite Antriebsanordnung eine flexible Antriebswelle (108) und ein an der flexiblen Antriebswelle befestigtes Eingangszahnrad (120) aufweist; und
eine dritte Antriebsanordnung (50), die Folgendes aufweist:
einen hydraulischen Zylinder (550), der in dem flexiblen Außenschlauch angeordnet ist; und
einen ersten Kolben (520), der innerhalb des hydraulischen Zylinders bewegbar ist; und
eine Werkzeuganordnung (16), die an einem distalen Abschnitt des flexiblen Außenschlauchs befestigt ist, wobei die Werkzeuganordnung Folgendes aufweist:
eine Ambossanordnung (20), die einen Ambosskopf (130) und eine am Ambosskopf befestigte Ambosswelle (132) aufweist; und
eine Schalenbaugruppe nach Anspruch 1,
wobei das Klemmzahnrad innerhalb des Gehäuses der Schalenbaugruppe getragen wird, wobei das Klemmzahnrad funktionsfähig mit dem Eingangszahnrad der zweiten Antriebsanordnung in Eingriff steht;
wobei durch Aktivieren der ersten Antriebsanordnung durch eine erste Klemmstufe das flexible Artikulationsglied zurückgezogen wird, um den Ambosshalter zum Bewegen der Ambossanordnung aus einer geöffneten Position in eine teilweise geklemmte Position, in der der Ambosshalter funktionsfähig mit dem Klemmzahnrad in Eingriff steht, proximal zu bewegen,
wobei durch Aktivieren der zweiten Antriebsanordnung durch eine zweite Klemmstufe der Ambosshalter weiter proximal bewegt wird, um die Ambossanordnung aus der teilweise geklemmten Position in eine vollständig geklemmte Position zu bewegen, und
durch Aktivieren der dritten Antriebsanordnung der erste Kolben vorgeschoben wird, um das Fluid in das Gehäuse der Schalenbaugruppe zum Vorschieben des zweiten Kolbens zu leiten, wodurch die Schieberanordnung vorgeschoben wird.

9. Chirurgische Klammernahtvorrichtung nach Anspruch 8, wobei die Ambosswelle einen äußeren Gewindeabschnitt aufweist, der mit dem Klemmzahnrad deckungsgleich ist, wenn sich die Ambossanordnung in der teilweise zurückgezogenen Position befindet.

10. Chirurgische Klammernahtvorrichtung nach Anspruch 9, wobei die Ambosswelle lösbar an den Ambosshalter gekoppelt ist.

11. Chirurgische Klammernahtvorrichtung nach Anspruch 8, ferner eine Griffanordnung (12) aufweisend, wobei die Adapteranordnung einen proximalen Abschnitt aufweist, der an die Griffanordnung gekoppelt ist.

12. Chirurgische Klammernahtvorrichtung nach Anspruch 8, wobei die Adapteranordnung einen flexiblen Fluidzuführungsschlauch (560) aufweist, der den hydraulischen Zylinder und das Gehäuse der Schalenbaugruppe miteinander verbindet, so dass der hydraulische Zylinder und das Gehäuse der Schalenbaugruppe in Fluidverbindung sind.

13. Chirurgische Klammernahtvorrichtung, umfassend:
eine Adapteranordnung (14), die Folgendes aufweist:
einen flexiblen Außenschlauch (32);
eine erste Antriebsanordnung (36), die sich durch den flexiblen Außenschlauch erstreckt, wobei die erste Antriebsanordnung ein flexibles Annäherungsglied (84) und einen an einem distalen Abschnitt des flexiblen Annäherungsglieds befestigten Ambosshalter (88) aufweist;
eine zweite Antriebsanordnung (40), die sich durch den flexiblen Außenschlauch erstreckt, wobei die zweite Antriebsanordnung eine flexible Antriebswelle (108) und ein an einem distalen Abschnitt der flexiblen Antriebswelle befestigtes Eingangszahnrad (120) aufweist; und
eine dritte Antriebsanordnung (50), die einen hydraulischen Zylinder (550), einen ersten Kolben (520) und
einen flexiblen Schlauch (560) in Fluidverbindung mit dem hydraulischen Zylinder aufweist; und
eine Werkzeuganordnung (16), die an einem distalen Abschnitt des flexiblen Außenschlauchs befestigt ist, wobei die Werkzeuganordnung Folgendes aufweist:
eine Ambossanordnung (20), die einen Ambosskopf (130) und eine am Ambosskopf befestigte Ambosswelle (132) aufweist; und
eine Schalenbaugruppe nach Anspruch 1, wobei der Hohlraum mit dem flexiblen Schlauch der dritten Antriebsanordnung in Fluidverbindung steht; und
das Klemmzahnrad mit dem Eingangszahnrad der zweiten Antriebsanordnung in Eingriff steht,
wobei durch Aktivieren der ersten Antriebsanordnung durch eine erste Klemmstufe das flexible Annäherungsglied zurückgezogen wird, um den Ambosshalter zum Bewegen der Ambossanordnung aus einer geöffneten Position in eine teilweise geklemmte Position, in der die Ambosswelle funktionsfähig mit dem Klemmzahnrad in Eingriff steht, proximal zu bewegen,
wobei durch Aktivieren der zweiten Antriebsanordnung durch eine zweite Klemmstufe der Ambosshalter weiter proximal bewegt wird, um die Ambossanordnung aus der teilweise geklemmten Position in eine vollständig geklemmte Position zu bewegen, und
wobei durch Aktivieren der dritten Antriebsanordnung der erste Kolben im hydraulischen Zylinder vorgeschoben wird, um ein Fluid in den Hohlraum des Schalengehäuses durch den flexiblen Schlauch zu leiten, wodurch die Schieberanordnung vorgeschoben wird.

14. Chirurgische Klammernahtvorrichtung nach Anspruch 13, wobei die Werkzeuganordnung ferner ein ringförmiges Messer (310) aufweist, das auf dem hydraulischen Kolben getragen wird.

15. Chirurgische Klammernahtvorrichtung nach Anspruch 14, wobei das Klemmzahnrad eine Gewindebohrung aufweist, die ausgelegt ist, einen äußeren Gewindeabschnitt der Ambosswelle in Gewindeeingriff zu nehmen.

## Revendications

1. Ensemble coque (18) comprenant :
un logement de coque (156) définissant une cavité (174) ;
une pluralité d'agrafes (206) ;
une cartouche d'agrafes (202) supportée sur le logement de coque, la cartouche d'agrafes définissant des fentes (204) qui reçoivent la pluralité d'agrafes ;
un ensemble de poussoirs (212) positionné à l'intérieur de la cavité du logement de coque et comportant une pluralité de poussoirs (212a) disposés en une configuration annulaire pour éjecter la pluralité d'agrafes de la cartouche d'agrafes ; et
un piston hydraulique (500) configuré pour entrer en contact avec l'ensemble de poussoirs pour communiquer un déplacement axial à l'ensemble de poussoirs et former un joint étanche contre le logement de coque,
dans lequel l'alimentation en fluide dans la cavité du logement de coque fait avancer le piston hydraulique, qui, à son tour, fait avancer l'ensemble de poussoirs pour éjecter la pluralité d'agrafes de la cartouche d'agrafes, dans lequel le logement de coque comprend un guide annulaire (201) dans la cavité, le guide annulaire définissant un alésage (159a) à travers celui-ci ;
**caractérisé en ce que** le logement de coque comporte un engrenage de serrage (168) supporté de manière rotative dans celui-ci.

2. Ensemble coque selon la revendication 1, comprenant en outre un couteau annulaire (310) supporté sur le piston hydraulique pour un déplacement axial concomitant avec celui-ci.

3. Ensemble coque selon la revendication 1 ou 2, dans lequel le piston hydraulique définit des rainures circonférentielles interne et externe (506, 507) qui reçoivent des joints toriques interne et externe (512, 514) respectifs.

4. Ensemble coque selon la revendication 3, dans lequel le joint torique interne est configuré pour venir en prise avec le guide annulaire du logement de coque.

5. Ensemble coque selon la revendication 1, dans lequel l'engrenage de serrage est aligné sur le guide annulaire du logement de coque.

6. Ensemble coque selon la revendication 5, dans lequel une partie proximale du logement de coque comporte une lèvre interne (157) définissant une découpe pour supporter de manière rotative un engrenage d'entrée de serrage (120) de telle sorte que l'engrenage d'entrée de serrage entre en prise fonctionnelle avec l'engrenage de serrage.

7. Ensemble coque selon la revendication 6, dans lequel la lèvre interne définit un alésage en communication avec la cavité du logement de coque.

8. Dispositif d'agrafage chirurgical comprenant :
un ensemble adaptateur (14) comportant :
un tube externe flexible (32) ;
un premier ensemble d'entraînement (36) s'étendant à travers le tube externe flexible, le premier ensemble d'entraînement comportant une liaison de rapprochement flexible (84) et un élément de retenue d'enclume (88) fixé à la liaison de rapprochement flexible ;
un deuxième ensemble d'entraînement (40) s'étendant à travers le tube externe flexible, le deuxième ensemble d'entraînement comportant un arbre d'entraînement flexible (108) et un engrenage d'entrée (120) fixé à l'arbre d'entraînement flexible ; et
un troisième ensemble d'entraînement (50) comportant :
un vérin hydraulique (550) disposé dans le tube externe flexible ; et
un premier piston (520) mobile à l'intérieur du vérin hydraulique ; et
un ensemble d'outils (16) fixé à une partie distale du tube externe flexible, l'ensemble d'outils comportant :
un ensemble enclume (20) comportant une tête d'enclume (130) et une tige d'enclume (132) fixée à la tête d'enclume ; et
une ensemble coque selon la revendication 1,
dans lequel l'engrenage de serrage est supporté à l'intérieur du logement de l'ensemble coque, l'engrenage de serrage est en prise fonctionnelle avec l'engrenage d'entrée du deuxième ensemble d'entraînement ;
dans lequel l'activation du premier ensemble d'entraînement au cours d'une première phase de serrage rétracte la liaison d'articulation flexible pour déplacer l'élément de retenue d'enclume de façon proximale pour déplacer l'ensemble enclume d'une position ouverte à une position partiellement serrée dans laquelle l'élément de retenue d'enclume est en prise fonctionnelle avec l'engrenage de serrage,
l'activation du deuxième ensemble d'entraînement au cours d'une seconde phase de serrage déplace l'élément de retenue d'enclume plus loin de manière proximale pour déplacer l'ensemble enclume de la position partiellement serrée à une position complètement serrée, et
l'activation du troisième ensemble d'entraînement fait avancer le premier piston pour diriger le fluide dans le logement de l'ensemble coque pour faire avancer le second piston, ce qui fait avancer l'ensemble de poussoirs.

9. Dispositif d'agrafage chirurgical selon la revendication 8, dans lequel la tige d'enclume a une partie externe filetée alignée sur l'engrenage de serrage lorsque l'ensemble enclume est dans la position partiellement rétractée.

10. Dispositif d'agrafage chirurgical selon la revendication 9, dans lequel la tige d'enclume est accouplée de manière amovible à l'élément de retenue d'enclume.

11. Dispositif d'agrafage chirurgical selon la revendication 8, comportant en outre un ensemble poignée (12), l'ensemble adaptateur ayant une partie proximale accouplée à l'ensemble poignée.

12. Dispositif d'agrafage chirurgical selon la revendication 8, dans lequel l'ensemble adaptateur comporte un tube flexible d'alimentation en fluide (560) interconnectant le vérin hydraulique et le logement de l'ensemble coque de telle sorte que le vérin hydraulique et le logement de l'ensemble coque soient en communication fluidique.

13. Dispositif d'agrafage chirurgical comprenant :
un ensemble adaptateur (14) comportant :
un tube externe flexible (32) ;
un premier ensemble d'entraînement (36) s'étendant à travers le tube externe flexible, le premier ensemble d'entraînement comportant une liaison de rapprochement flexible (84) et un élément de retenue d'enclume (88) fixé à une partie distale de la liaison de rapprochement flexible ;
un deuxième ensemble d'entraînement (40) s'étendant à travers le tube externe flexible, le deuxième ensemble d'entraînement comportant un arbre d'entraînement flexible (108) et un engrenage d'entrée (120) fixé à une partie distale de l'arbre d'entraînement flexible ; et
un troisième ensemble d'entraînement (50) comportant un vérin hydraulique (550), un premier piston (520), et
un tube flexible (560) en communication fluidique avec le vérin hydraulique ; et
un ensemble d'outils (16) fixé à une partie distale du tube externe flexible, l'ensemble d'outils comportant :
un ensemble enclume (20) comportant une tête d'enclume (130) et une tige d'enclume (132) fixée à la tête d'enclume ; et
un ensemble coque selon la revendication 1, dans lequel la cavité est en communication fluidique avec le tube flexible du troisième ensemble d'entraînement ; et
l'engrenage de serrage est en prise avec l'engrenage d'entrée du deuxième ensemble d'entraînement,
dans lequel l'activation du premier ensemble d'entraînement au cours d'une première phase de serrage rétracte la liaison de rapprochement flexible pour déplacer l'élément de retenue d'enclume de façon proximale pour déplacer l'ensemble enclume d'une position ouverte à une position partiellement serrée, dans laquelle la tige d'enclume est en prise fonctionnelle avec l'engrenage de serrage,
dans lequel l'activation du deuxième ensemble d'entraînement au cours d'une seconde phase de serrage déplace l'élément de retenue d'enclume plus loin de manière proximale pour déplacer l'ensemble enclume de la position partiellement serrée à une position complètement serrée, et
dans lequel l'activation du troisième ensemble d'entraînement fait avancer le premier piston dans le vérin hydraulique pour diriger un fluide dans la cavité du logement de coque à travers le tube flexible, faisant de ce fait avancer l'ensemble de poussoirs.

14. Dispositif d'agrafage selon la revendication 13, dans lequel l'ensemble d'outils comprend en outre un couteau annulaire (310) supporté sur le piston hydraulique.

15. Dispositif d'agrafage selon la revendication 14, dans lequel l'engrenage de serrage a un alésage fileté configuré pour entrer en prise par vissage avec une partie externe filetée de la tige d'enclume.
